(19) Europäisches Patentamt
European Patent Office
Office européen des brevets

(11) Veröffentlichungsnummer: **0 003 344**
**B1**

(12) **EUROPÄISCHE PATENTSCHRIFT**

(45) Veröffentlichungstag der Patentschrift: **15.04.81**

(21) Anmeldenummer: **79100182.9**

(22) Anmeldetag: **22.01.79**

(51) Int. Cl.³: **C 07 C 79/12, C 07 C 121/52**

# (54) Verfahren zur Herstellung von Fluorbenzolen.

(30) Priorität: **26.01.78 DE 2803259**

(43) Veröffentlichungstag der Anmeldung: **08.08.79 Patentblatt 79/16**

(45) Bekanntmachung des Hinweises auf die Patenterteilung: **15.04.81 Patentblatt 81/15**

(84) Benannte Vertragsstaaten: **BE CH DE FR GB**

(56) Entgegenhaltungen:
**GB - 1 360 327**
**US - A - 4 069 262**

(73) Patentinhaber: **BASF Aktiengesellschaft**
**Carl-Bosch-Strasse 38**
**D-6700 Ludwigshafen (DE)**

(72) Erfinder: **Oeser, Heinz-Guenter, Dr.**
**Friedrich-Burschell-Weg 19**
**D-6700 Ludwigshafen (DE)**
Erfinder: **Koenig, Karl-Heinz, Dr.**
**Pierstrasse 8 a**
**D-6710 Frankenthal (DE)**
Erfinder: **Mangold, Dietrich, Dr.**
**Hermann-Walker-Strasse 49**
**D-6903 Neckargemuend (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 344 B1

Verfahren zur Herstellung von Fluorbenzolen

Die Erfindung betrifft ein neues Verfahren zur Herstellung von Fluorbenzolen durch Umsetzung von Chlorbenzolen mit Kaliumfluorid in Gegenwart von Kronenäther und von Lösungsmitteln.

In Chemistry and Industry 1962, Seiten 1 328 und 1 329, wird die Herstellung von o- und p-Fluornitrobenzol durch Umsetzung von entsprechenden Chlorbenzolen mit Kaliumfluorid bei 221 bis 230°C während 7 Stunden unter Verwendung von Dimethylsulfon mit einer Ausbeute von 60 bis 73 Prozent beschrieben. Es wird darauf hingewiesen, daß in Dimethylformamid oder Dimethylsulfoxid die Fluorierung meist nur träge erfolgt und die Umsetzung unbefriedigend ist. In Dimethylsulfoxid treten Zersetzungen des Reaktionsgemisches unter Bildung von übelriechenden, schwefelhaltigen Nebenprodukten auf; Reaktionszeiten von 15 Stunden werden angegeben.

J. Am. Chem. Soc., Band 78 (1956), Seiten 6 034 bis 6 036, beschreibt die Umsetzung verschiedener Mononitrochlorbenzole in Dimethylformamid und Dimethylsulfoxid mit Ausbeuten zwischen 10 und 72 Prozent, Reaktionszeiten von 2 bis 163 Stunden und bei Temperaturen von 175 bis 190°C.

Die Umsetzung von 2-Chlornitrobenzol in Dimethylformamid ergab trotz einer Umsetzung während 163 Stunden bei 170°C nur eine geschätzte Ausbeute von 40 Prozent.

Es ist aus den Chemical Abstracts, Band 57 (1962), Seite 9 706 h bis 9 707 b bekannt, daß man anstelle von Kaliumfluorid die Umsetzung auch mit Caesiumfluorid bei 190 bis 200°C in Abwesenheit von Lösungsmitteln und mit einer Reaktionszeit von 25 Stunden durchführen kann; ein Überschuß von 30 bis 50 Prozent Caesiumfluorid über die stöchiometrische Menge ist notwendig. Man erhält o-Fluornitrobenzol mit 80 bis 8 Prozent Ausbeute, die p-Isomere mit 70 bis 80 Prozent Ausbeute.

Nach den Angaben der GB-Patentschrift 1 360 327 nimmt man die Umsetzung von Chlornitrobenzol mit Kaliumfluorid in Dimethylacetamid als Lösungsmittel vor. Die US-Patentschrift 3 064 058 beschreibt eine Umsetzung in Tetramethylensulfon als Lösungsmittel. Um ein trockenes Reaktionsgemisch zu erhalten, wird das Verfahren so durchgeführt, daß man zuerst das Alkalifluorid in dem Lösungsmittel suspendiert und dann die Suspension so lange destilliert, bis das gesamte in der Suspension anwesende Wasser zusammen mit 5 bis 15 Gewichtsprozent Tetramethylensulfon abgetrennt ist. Anschließend wird p-Nitrochlorbenzol der Suspension zugesetzt. Als Alkalifluorid werden nur Natriumfluorid und Kaliumfluorid erwähnt und in den Beispielen allein Kaliumfluorid beschrieben. Wie die Beispiele zeigen, wird die Ausbeute von 93,5 Prozent bei einer Reaktionstemperatur von 240°C und mit einer Reaktionszeit von 12 Stunden erzielt, hinzu kommt die zusätzliche Zeit der Destillation.

Die US-Patentschrift 3 240 824 zieht aus dem vorgenannten Stand der Technik den Schluß, daß Mononitro-o- bzw. -p-chlorbenzole optimal mit Kaliumfluorid als einzigem Alkalifluorid in Abwesenheit von Lösungsmitteln bei einer Temperatur von 270 bis 320°C umgesetzt werden. Es wird darauf hingewiesen, daß Caesiumfluorid zu unwirtschaftlich ist, auf der anderen Seite Lösungsmittel umständlich zurückgewonnen werden müssen, wobei Verluste auftreten; die Trocknung des lösungsmittelhaltigen Ausgangsgemisches ist häufig schwierig und bedarf besonderer Fraktioniertechnik. Die von der US-Patentschrift vorgeschlagenen Bedingungen liefern, im Hinblick auf alle Beispiele, bei Reaktionszeiten von 24 Stunden nur Ausbeuten von 56,5 Prozent (o-Verbindung) oder 60,6 Prozent (p-Verbindung) bzw. eine Umsetzung von 50,5 Prozent (o-Verbindung) bzw. 52,5 Prozent (p-Verbindung).

In J. Am Soc., Band 96 (1974), Seiten 2 250 bis 2 252, wird die Umsetzung von 2,4-Dinitrochlorbenzol mit Kaliumfluorid in Gegenwart von 18-Krone-6 beschrieben.

Aus der US—PS 4 069 262 ist bekannt, daß man 2-Fluornitrobenzol aus 2-Chlornitrobenzol und Kaliumfluorid herstellen kann, wenn man die Umsetzung bei Temperaturen von 240 bis 250°C in Kroneäther und Sulfolan durchführt. Die Ausbeuten sind mäßig; so ergab die Nacharbeitung von Beispiel 2 o-Fluornitrobenzol in einer Ausbeute von nur 40% d. Th. über eine Umsetzung bei Temperaturen unter 240°C wird in der US-Patentschrift mitgeteilt (s. Spalte 2, Zeilen 46 bis 49), daß dabei keine befriedigende Umwandlung erreicht wird. Es wurde nun gefunden, daß man Fluorbenzole der Formel

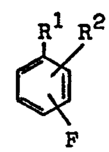

I,

worin $R^1$ eine Nitrogruppe oder Cyangruppe bedeutet, $R^2$ ein Wasserstoffatom, einen aliphatischen Rest oder ein Halogenatom bezeichnet, durch Umsetzung von Chlorbenzolen mit Kaliumfluorid in Gegenwart von Lösungsmitteln vorteilhaft erhält, wenn man Chlorbenzole der Formel

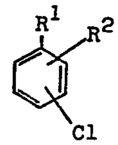

II,

worin $R^1$ und $R^2$ die vorgenannte Bedeutung besitzen, in Gegenwart von Kronenäther und in Gegenwart von N,N-disubstituierten Carbonsäureamiden, Nitrobenzol, Nitrilen, aliphatischen Sulfonen und/oder aliphatischen Sulfoxiden als Lösungsmitteln bei Temperaturen von 160 bis 200°C umsetzt.

Die Umsetzung wird unter Verwendung von o-Chlornitrobenzol durch die folgenden Formeln wiedergegeben:

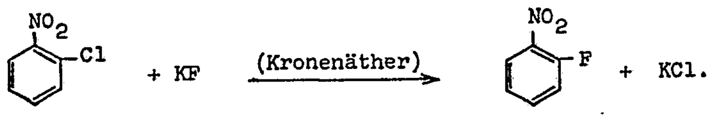

Im Vergleich mit dem Stand der Technik liefert das Verfahren nach der Erfindung ein optimales Ergebnis mit Bezug auf Ausbeute, Reinheit des Endstoffs, niedrige Reaktionstemperatur und nahezu vollständige Lösungsmittelrückführung. Gegenüber fast allen Verfahren ist die Ausbeute und Reinheit besser. Im Vergleich zu dem in Chemical Abstracts, Band 57 (loc. cit.), beschriebenen Verfahren ist das erfindungsgemäße Verfahren wirtschaftlicher und auch im großtechnischen Maßstab durchführbar. Gegenüber dem in der US-Patentschrift 3 064 058 beschriebenen Verfahren ist eine einfache Trocknung des Kaliumfluorids, z.B. während 1,5 bis 2,5 Stunden in Trockenschränken, ausreichend und man erspart die umständliche, azeotrope Entfernung des Wassers und damit Verluste an Betriebszeit, Lösungsmittel und Energie. Dieses vorteilhafte Verfahrensergebnis ist z.B auch im Hinblick auf die in J. Am. Soc., Band 96 (1974), Seiten 2 250 bis 2 252 beschriebene Umsetzung von 2,4-Dinitrochlorbenzol mit Kaliumfluorid in Gegenwart von 18-Krone-6 überraschend, da die nach der Erfindung zu verwendenden Ausgangsstoffe, welche nur eine Nitrogruppe aufweisen, bisher nur bei sehr hohen Temperaturen und mit mäßigen Ausbeuten in die entsprechenden Fluorverbindungen übergeführt werden konnten und unter den Bedingungen des bekannten Verfahrens, d.h. in Gegenwart von 18-Krone-6 bei Temperaturen von 83°C, keine brauchbaren Resultate ergeben. Nach den Angaben der US-Patentschrift 4 069 262 konnte nicht erwartet werden, daß das erfindungsgemäße Verfahren, das bei Temperaturen von 160 bis 200°C durch-geführt wird, so vorteilhafte Ergebnisse liefert.

Der Ausgangsstoff II wird mit Kaliumfluorid in stöchiometrischer Menge oder im Überschuß, vorzugsweise in einer Menge von 1 bis 5, vorzugsweise von 2 bis 4,5 Mol Kaliumfluorid je Mol Ausgangsstoff II umgesetzt. Bevorzugte Ausgangsstoffe II und dementsprechend bevorzugte Endstoffe I sind solche, in deren Formeln $R^1$ eine Nitrogruppe oder Cyangruppe bedeutet und $R^2$ ein Wasserstoffatom, einen Alkylrest mit 1 bis 4 Kohlenstoffatomen, der noch durch unter den Reaktionsbedingungen inerte Gruppen, z.B. Alkylgruppen oder Alkoxygruppen mit 1 bis 4 Kohlenstoffatomen, substituiert sein kann, ein Bromatom, Jodatom oder insbesondere Fluoratom bezeichnet. Das Fluoratom des Endstoffs I bzw. das Chloratom des Ausgangsstoffs II kann in Metastellung, vorteilhaft in Parastellung oder insbesondere in Orthostellung zur Nitrogruppe bzw. Cyangruppe stehen.

Es kommen z.B. folgende Chlorbenzole II in Betracht: o-Chlornitrobenzol, m-Chlornitrobenzol, p-Chlornitrobenzol, o-Chlorbenzonitril, m-Chlorbenzonitril, p-Chlorbenzonitril; 2-Methyl-, 2-Äthyl-, 2-Propyl-, 2-Isopropyl-, 2-Butyl-, 2-Isobutyl-, 2-sek.-Butyl-, 2-tert.-Butyl, 2-Fluro-4-chlornitrobenzol, 4-Methyl-, 4-Äthyl-, 4-Propyl-, 4-Isopropyl-, 4-Butyl-, 4-Isobutyl-, 4-sek.-Butyl-, 4-tert.-Butyl-, 4-Fluor-2-chlornitrobenzol und entsprechend anstelle in 4-Stellung in 3-Stellung, 5-Stellung. 6-Stellung substituierte 2-Chlornitrobenzole; entsprechend substituierte 2-Chlorbenzonitrile und 4-Chlorbenzonitrile, 3-Chlornitrobenzole und 3-Chlorbenzonitrile.

Von besonderem Interesse ist die Herstellung von Fluorbenzolen der Formel

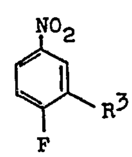

III,

in der $R^3$ einen aliphatischen Rest oder ein Halogenatom bezeichnet, aus den Chlorbenzolen der Formel

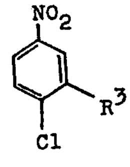

IV,

in der $R^3$ die obengenannte Bedeutung hat. Chlorbenzole der Formel IV und z.B. 3,4-Dichlornitrobenzol, 3-Methyl-4-chlornitrobenzol und 3-Fluor-4-chlornitrobenzol.

Als Kronenäther kommen z.B. 18-Krone-6, 15-Krone-5 und Dibenzo-18-Krone-6 in Betracht. Man

verwendet den Kronenäther zweckmäßig in einer Menge unterhalb 0,1 vorteilhaft zwischen 0,001 und 0,1, bevorzugt von 0,003 bis 0,03, insbesondere von 0,05 bis 0,025 Mol Kronenäther je Mol Ausgangsstoff II. Geeignete Lösungsmittel sind beispielsweise N,N-disubstituierte Carbonamide wie Dimethylformamid, Tetramethylharnstoff; Nitrobenzol; Nitrile wie Acetonitril, Benzonitril; bevorzugt sind Dimethylformamid und insbesondere aliphatische Sulfone und Sulfoxide, vorteilhaft der Formel.

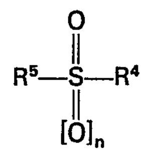

V,

worin $R^4$ und $R^5$ gleich oder verschieden sind und jeweils einen aliphatischen Rest, vorzugsweise einen Alkylrest mit 1 bis 8, insbesondere 1 bis 4 Kohlenstoffatomen oder $R^4$ und $R^5$ zusammen einen Alkylenrest mit 4 oder 5 Kohlenstoffatomen bedeuten und n 0 oder 1 bezeichnet. Geeignete Lösungsmittel V sind z.B. Dimethylsulfoxid, Diäthylsulfoxid, Dipropylsulfoxid, Diisopropylsulfoxid, Di-n-butylsulfoxid, Diisobutylsulfoxid, Dipentylsulfoxid, Dihexylsulfoxid, Diheptylsulfoxid, Dioctylsulfoxid, Methyläthylsulfoxid, Tetramethylensulfoxid, Pentamethylensulfoxid, Dimethylsulfon, Diäthylsulfon, Dipropylsulfon, Diisopropylsulfon, Dibutylsulfon, Diisobutylsulfon, Dipentylsulfon, Dihexylsulfon, Diheptylsulfon, Dioctylsulfon, Methyläthylsulfon, Tetramethylensulfon, Pentamethylensulfon; besonders bevorzugt ist Tetramethylensulfon. Auch Lösungsmittelgemische können verwendet werden. Zweckmäßig verwendet man das Lösungsmittel in einer Menge von 50 bis 1 000 Gewichtsprozent, vorzugsweise von 100 bis 200 Gewichtsprozent, bezogen auf Ausgangsstoff II.

Die Umsetzung wird bei einer Temperatur von 160 bis 200°C drucklos oder unter Druck, kontinuierlich oder diskontinuierlich durchgeführt.

Die Reaktion kann wie folgt durchgeführt werden: Ein Gemisch von Ausgangsstoff II, Kaliumfluorid, Kronenäther und Lösungsmittel wird während 2 bis 100 Stunden, bei der Reaktionstemperatur gehalten. Das Kaliumfluorid wird vor der Reaktion auf beliebige Weise getrocknet; zweckmäßig erfolgt die Trocknung auf berieblich einfachem Wege, z.B. in Trockenkammern oder in Kammertrocknern, Spiralbandtrocknern oder Hordentrocknern; man kann aber auch Wirbelschichttrockner, Trommeltrockner oder Tellertrockner verwenden. Nach der Umsetzung wird der Endstoff auf übliche Weise, z.B. durch Filtration, Waschen des Festgutes und Destillation von Filtrat und Waschfiltraten, isoliert.

Die nach dem Verfahren der Erfindung herstellbaren Fluorbenzole sind wertvolle Ausgangsstoffe für die Herstellung von Farbstoffen, Pharmazeutika und Schädlingsbekämpfungsmitteln. So kann man durch Reduktion Fluoraniline herstellen, die Verwendung für Anstrichfarben, Germicide oder Lötflußmittel finden. Bezüglich der Verwendung wird auf die vorgenannten Veröffentlichungen verwiesen.

Die in den folgenden Beispielen aufgeführten Teile bedeuten Gewichtsteile.

## Beispiel 1

1 300 Teile o-Chlornitrobenzol, 960 Teile Kaliumfluorid und 82,5 Teile 18-Krone-6 werden unter Stickstoff in 1 300 Teilen Tetramethylensulfon 30 Stunden bei 180°C gerührt. Die auf 110°C abgekühlte Lösung wird abgesaugt, das Festgut mit 500 Teilen Dichlormethan nachgewaschen, die Filtrate vereinigt und destilliert. Man erhält 932 Teile (80% der Theorie) o-Fluornitrobenzol vom Kp 55 bis 57°C (1,2 Torr) sowie 1 290 Teile (99,2% d.Th.) Tetramethylensulfon vom Kp 105 bis 110°C. Aus dem Destillationsrückstand kann durch mehrmaliges Ausschütteln mit Wasser der Kronenäther zu ca. 70% zurückgewonnen werden.

## Beispiel 2

Eine Suspension von 1 500 Teilen 3,4-Dichlornitrobenzol, 680 Teilen Kaliumfluorid und 25 Teilen 18-Krone-6 in 1 420 Teilen Dimethylformamid wird 9 Stunden unter Rückfluß (165°C) gekocht. Die Aufarbeitung erfolgt analog Beispiel 1. Man erhält 1 124 Teile (82% d. Th.) 3-Chlor-4-fluornitrobenzol vom Fp. 41 bis 42°C.

## Beispiel 3

1 500 Teile 3,4-Dichlornitrobenzol, 680 Teile Kaliumfluorid und 20 Teile 18-Krone-6 werden unter Stickstoff in 1 300 Teilen Tetramethylensulfon 4 Stunden bei 180°C gerührt. Die Aufarbeitung erfolgt analog Beispiel 1. Man erhält:

1. Fraktion: $Kp_{0,5}$: 63—65°C
A: 1203 g (87,8%) 3-Chlor-4-fluornitrobenzol

2. Fraktion: $Kp_{0,5}$: 65—100°C
A: 80 g (40,4% 3-Chlor-4-fluornitrobenzol 39,8% 3,4-Dichlornitrobenzol und 19.8% Sulfolan)

3. Fraktion: $Kp_{0,5}$: 100°C
A: 1256 g (96,6%) Sulfolan.

**Patentanspruch**

Verfahren zur Herstellung von Fluorbenzolen der Formel

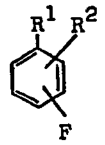

I,

worin $R^1$ eine Nitrogruppe oder Cyangruppe bedeutet, $R^2$ ein Wasserstoffatom, einen aliphatischen Rest oder ein Halogenatom bezeichnet, durch Umsetzung von Chlorbenzolen mit Kaliumfluorid in Gegenwart von Lösungsmitteln dadurch gekennzeichnet, daß man Chlorbenzole der Formel

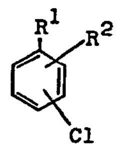

II,

worin $R^1$ und $R^2$ die vorgenannte Bedeutung besitzen, in Gegenwart von Kronenäther und in Gegenwart von N,N-disubstituierten Carbonsäureamiden, Nitrobenzol, Nitrilen, aliphatischen Sulfonen und/oder aliphatischen Sulfoxiden als Lösungsmittel bei Temperaturen von 160 bis 200°C umsetzt.

**Revendication**

Procédé pour préparer des fluorobenzènes de formule

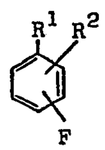

I,

dans laquelle $R^1$ représente un groupe nitro ou un groupe cyano, $R^2$ représente un atome d'hydrogène, un reste aliphatique ou un atome d'halogène, par réaction de chlorobenzène avec du fluorure de potassium en présence de solvants, caractérisé en ce que l'on fait réagir des chlorobenzènes de formule

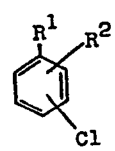

II,

dans laquelle $R^1$ et $R^2$ ont des significations indiquées cidessus, en présence d'éthers en couronne et en présence de carboxamides disubstitués à l'azote, de nitrobenzène, de nitrile, de sulfones aliphatiques et/ou de sulfoxydes aliphatiques servant de solvants, à des températures de 160 à 200°C.

**Claim**

A process for the preparation of a fluorobenzene of the formula

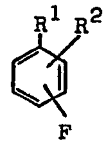

I,

where $R^1$ is nitro or cyano and $R^2$ is hydrogen, an aliphatic radical or halogen, by reaction of a chlorobenzene with potassium fluoride in the presence of a solvent, characterized in that a chlorobenzene of the formula

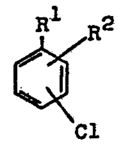

II,

where $R^1$ and $R^2$ have the above meanings, is reacted in the presence of a crown ether and of an N,N-disubstituted carboxylic acid amide, nitrobenzene, a nitrile, an aliphatic sulfone and/or an aliphatic sulfoxide as the solvent, at from 160 to 200°C.